# FASCICULE DE BREVET EUROPEEN

(11) **EP 1 795 136 B1**
(45) Date de publication et mention de la délivrance du brevet: **06.05.2009**
(21) Numéro de dépôt: 06356138.5
(22) Date de dépôt: 06.12.2006
(51) Int. Cl.: A61B 17/70, A61B 17/80, A61B 17/86

(54) **Dispositif de stabilisation dynamique du rachis**
Einrichtung zur dynamischen Stabilisierung von Wirbeln
Apparatus for dynamic stabilisation of the spine

(30) Priorité: 07.12.2005 FR 0512428
(43) Date de publication de la demande: 13.06.2007
(73) Titulaire: PHUSIS, 38330 Saint Ismier (FR)
(72) Inventeur: Tornier, Alain, 38330 St. Ismier (FR)
(74) Mandataire: Myon, Gérard Jean-Pierre

(56) Documents cités:
- WO-A-03/063714
- WO-A-03/094699
- US-A1- 2004 158 250
- US-B1- 6 328 738

## Description

La présente invention concerne un dispositif de stabilisation dynamique du rachis, destiné à être implanté le long de la colonne vertébrale en vue de stabiliser au moins deux vertèbres l'une par rapport à l'autre, tout en reproduisant une liaison intervertébrale articulaire. Une telle stabilisation est recherchée notamment dans le cadre du traitement du rachis dégénératif ou traumatique. L'invention s'intéresse plus particulièrement au traitement du rachis dorso-lombaire mais s'applique également au traitement du rachis cervical.

Pour traiter une instabilité intervertébrale, une première possibilité connue consiste à fusionner deux vertèbres adjacentes, ce qui revient à priver ces deux vertèbres de leur liberté de mouvements relatifs. Des montages totalement rigides sont à cet effet implantés de manière fixe le long du rachis pour bloquer définitivement la liaison articulaire entre les deux vertèbres à fusionner. Un exemple de ce genre de montages, à structure totalement figée en configuration d'implantation, est donné dans US-B-6,328,738. Cette intervention d'arthrodèse conduit cependant à une dégénérescence des disques adjacents sur laquelle il est nécessaire d'intervenir ultérieurement.

US-A-2004/0158250 propose également un montage destiné à être utilisé pour fusionner deux vertèbres adjacentes. Ce montage comporte deux éléments de plaque, qui sont fixés sur deux corps vertébraux et qui, juste après leur fixation, sont reliés par une liaison mécanique coulissante rectiligne. Cette mobilité n'est que très temporaire, dans le sens où le montage est rapidement figé dans sa totalité en raison de la colonisation de l'espace séparant les corps vertébraux par une greffe, étant remarqué que cette dernière risque d'être initialement mise en compression de manière excessive par les deux éléments de plaque.

Une autre possibilité connue de traitement du rachis consiste à intervenir à un stade plus précoce que celui impliquant une arthrodèse et vise à implanter un dispositif de stabilisation dynamique, comme proposé par exemple dans WO-A-03/094699. A cet effet, le dispositif comporte, d'une part, des vis d'ancrage osseux dans deux vertèbres immédiatement adjacentes, au niveau de leur pédicule, et, d'autre part, des éléments élastiques de liaison entre ces vis. Ces éléments souples, reliés rigidement à chaque vis, soulagent le disque intervertébral et corrigent d'éventuelles sur-pressions au niveau des surfaces articulaires entre ce disque et les vertèbres. Ces dispositifs offrent plus de confort au patient car ils permettent de conserver la mobilité du rachis. Cependant, en pratique, l'utilisation de ces dispositifs reliant les vertèbres de manière souple se révèle délicate : le dimensionnement de la flexibilité des éléments de liaison est difficile puisqu'il doit être adapté à chaque patient selon sa pathologie et sa morphologie et, à la longue, ces éléments risquent de voir leur comportement élastique évoluer. Le mauvais contrôle de ces paramètres ne permet pas de garantir le respect de la cinématique du rachis, ce qui peut conduire à une mauvaise stabilisation de la distance intervertébrale et à une aggravation des lésions que l'on cherche à traiter.

Le but de la présente invention est de proposer un dispositif de stabilisation dynamique du rachis reproduisant plus fidèlement le mouvement anatomique des vertèbres, plus efficace pour stabiliser les vertèbres traitées et plus fiable dans le temps.

A cet effet, l'invention a pour objet un dispositif de stabilisation dynamique du rachis destiné à reproduire une liaison intervertébrale articulaire, tel que défini à la revendication 1.

Ici, on entend par « configuration d'implantation » la configuration dans laquelle le dispositif est complètement implanté à des vertèbres du rachis, autrement dit après la fin de l'intervention chirurgicale d'implantation ce dispositif. Cette configuration d'implantation correspond donc à la configuration postopératoire du dispositif, après consolidation des vertèbres munies de ce dispositif.

L'utilisation des moyens de liaison rigides pour relier les ensembles vertébraux permet de conférer au dispositif un comportement cinématique stable dans le temps. Grâce à l'assemblage coulissant obtenu, ces moyens rigides imposent aux ensembles vertébraux des trajectoires de guidage pré-déterminées et fiables, garantissant que les mouvements articulaires intervertébraux sont efficacement centrés en une ou plusieurs zones pré-déterminées intervertébrales, prévues pour que ces comportements soient quasi-identiques ou, tout au moins, les plus proches possibles des comportements anatomiques normaux du rachis. De la sorte, en service, l'espace intervertébral est conservé, dans le sens où il n'est pas réduit, voire mis en compression par l'action dynamique du dispositif, car ce dernier encaisse les contraintes liées aux mouvements du rachis. En outre, l'implantation du dispositif selon l'invention se révèle facile puisque les mobilités internes au dispositif résident essentiellement, voire exclusivement, au niveau des liaisons coulissantes entre les ensembles vertébraux et les moyens mécaniques les reliant.

Une réalisation avantageuse de l'invention est définie à la revendication 2. Dans ce cas, le dispositif selon l'invention stabilise efficacement deux vertèbres immédiatement adjacentes, tout en leur garantissant une certaine mobilité essentiellement en flexion/extension, centrée sur l'espace discal inter-vertébral, c'est-à-dire une liberté de mouvements proche de la liberté anatomique normale. En effet, le dispositif supporte alors l'essentiel, voire la totalité des contraintes s'appliquant sur le disque intervertébral, en laissant à ce dernier sa mobilité.

Une structure particulièrement simple et efficace du dispositif selon l'invention est définie à la revendication 4.

D'autres caractéristiques avantageuses de ce dispositif, prises isolément ou suivant toutes les combinaisons techniquement possibles, sont énoncées aux revendications 3 et 5 à 14.

L'invention sera mieux comprise à la lecture de la description qui va suivre, donnée uniquement à titre d'exemple et faite en se référant aux dessins sur lesquels :
- les figures 1 et 2 sont des vues en élévation d'un premier mode de réalisation du dispositif selon l'invention, implanté au niveau de deux vertèbres, la figure 1 correspondant à une vue latérale de ces vertèbres tandis que la figure 2 correspond à une vue par l'arrière ;
- la figure 3 est une vue en élévation d'une partie du dispositif de la figure 1, dont certains composants sont représentés de manière éclatée ;
- la figure 4 est une coupe selon la ligne IV-IV de la figure 3 ;
- la figure 5 est une vue analogue à la figure 3 à plus grande échelle, illustrant une variante du premier mode de réalisation du dispositif selon l'invention ;
- la figure 6 est une vue en perspective et partielle d'une autre variante du premier mode de réalisation du dispositif selon l'invention ;
- la figure 7 est une vue analogue à la figure 3, illustrant une autre variante du premier mode de réalisation du dispositif selon l'invention ;
- la figure 8 est une vue en coupe selon la ligne VIII-VIII de la figure 7 ;
- les figures 9 et 10 sont des coupes d'une autre variante du premier mode de réalisation du dispositif selon l'invention, le plan de coupe de la figure 9 étant parallèle au plan sagittal des vertèbres tandis que le plan de la figure 10 est horizontal, le plan de la figure 10 étant référencé F₁₀-F₁₀ à la figure 9 ;
- les figures 11 à 13 concernent un second mode de réalisation du dispositif selon l'invention, la figure 11 correspondant à une vue de dessus du dispositif implanté au niveau d'une vertèbre, tandis que les figures 12 et 13 correspondent respectivement à des vues en élévation frontale par l'arrière et latérale de ce dispositif, analogues aux figures 2 et 1, certains composants du dispositif étant représentés de manière éclatée sur ces figures 11 à 13 ;
- la figure 14 est une vue analogue à la figure 1, illustrant une variante du premier mode de réalisation du dispositif selon l'invention, implantées au niveau de trois vertèbres adjacentes.

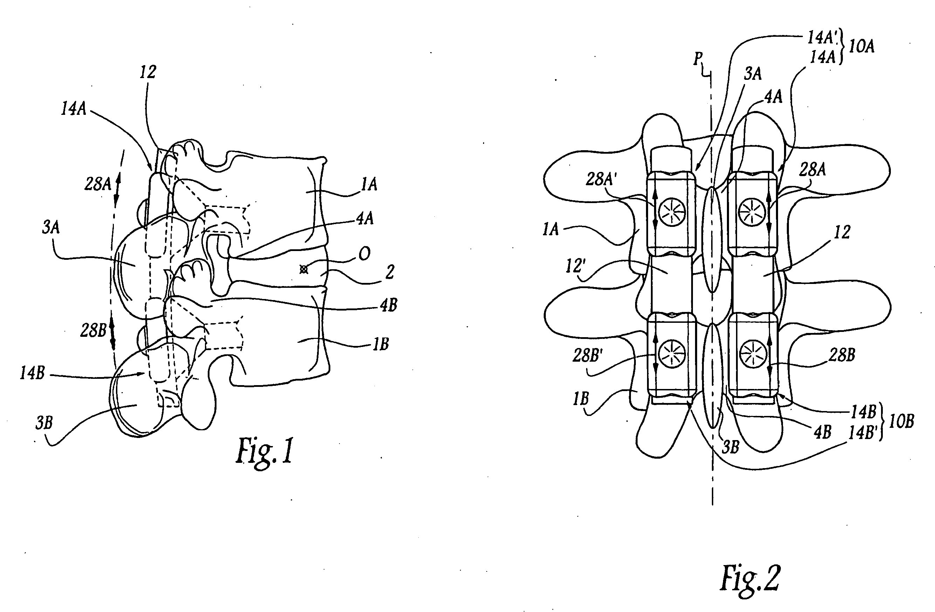

Sur les figures 1 et 2 sont représentées deux vertèbres adjacentes 1A et 1B d'un rachis lombaire d'un être humain. Ces vertèbres sont séparées l'une de l'autre par un disque intervertébral 2. Par commodité, la suite de la description est orientée par rapport à ces vertèbres dans leurs positions anatomiques, c'est-à-dire que les termes « postérieur », « arrière », « antérieur », « avant », « droit », « gauche », « supérieur », « inférieur », etc. s'entendent par rapport au rachis d'un patient se tenant debout.

Sur les figures 1 à 4 est représenté un dispositif de stabilisation des vertèbres 1A et 1B implanté sur le côté postérieur des vertèbres, en vue de reproduire la liaison articulaire entre ces vertèbres, tout en recréant l'espace intervertébral initial. Ce dispositif comporte essentiellement un ensemble vertébral 10A implanté au niveau de la vertèbre 1A, un ensemble vertébral 10B implanté au niveau de la vertèbre 1B et une paire de barres 12 et 12' reliant ces ensembles l'un à l'autre et s'étendant le long du rachis, comme détaillé ci-dessous.

Chaque ensemble vertébral 10A, 10B inclut à la fois un sous-ensemble vertébral droit 14A, 14B et un sous ensemble vertébral gauche 14A', 14B', disposés respectivement de part et d'autre du plan sagittal P contenant les apophyses épineuses 3A et 3B des vertèbres 1A et 1B. Les sous-ensembles droit 14A et 14B sont reliés mécaniquement par la barre 12 tandis que les sous-ensembles gauches 14A' et 14B' sont reliés mécaniquement par la barre 12'.

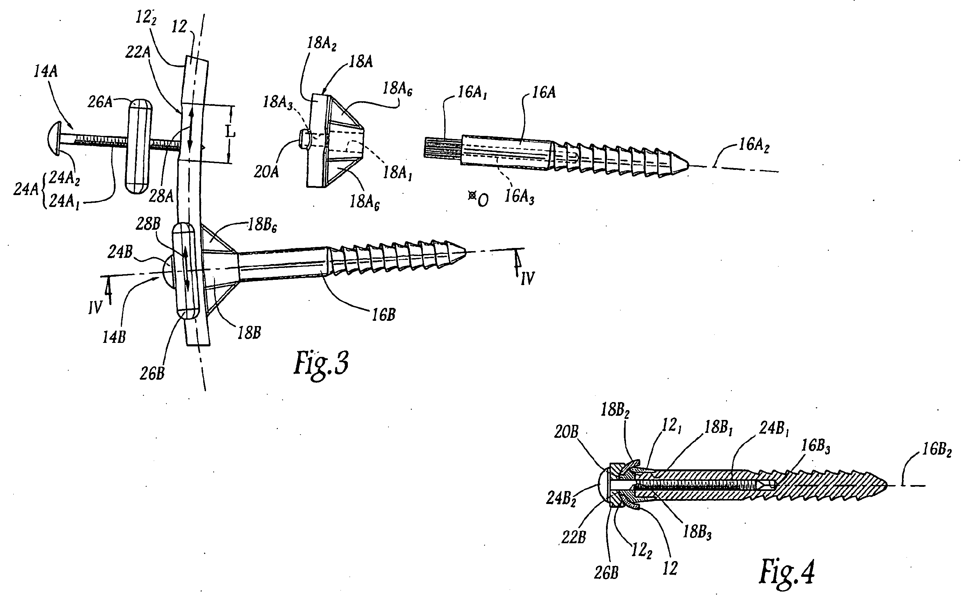

Chacun des sous-ensembles 14A, 14A', 14B et 14B' comporte des composants identiques, de sorte que, par commodité, seuls les composants visibles aux figures 3 et 4 seront décrits ci-après en détail, étant entendu que, par convention, et ce pour toute les formes de réalisation évoquées dans le présent document, les composants référencés avec la lettre « A » sont relatifs à la vertèbre 1A, tandis que les composants référencés avec la lettre « B » sont associés à la vertèbre 1B. De même, par convention, à la différence des composants droits, les composants gauches du dispositif sont référencés avec un prime. En outre, on remarquera que, globalement, les composants droits et gauches du dispositif sont implantés de manière symétrique par rapport au plan sagittal P du rachis passant pas les apophyses épineuses.

Comme représenté aux figures 3 et 4, chaque sous-ensemble 14A, 14B comporte une tige antérieure filetée 16A, 16B adaptée pour fixer le sous-ensemble aux vertèbres 1A, 1B. Chaque tige est dimensionnée pour s'ancrer fermement dans le pédicule 4A, 4B de la vertèbre, comme représenté aux figures 1 et 2.

A son extrémité postérieure, chaque tige 16A, 16B porte une tête mono-bloc 18A, 18B adaptée pour être solidarisée rigidement à la tige. A cet effet, chaque tête présente, à son extrémité antérieure, un logement 18A₁, 18B₁ de réception et d'immobilisation en rotation de l'extrémité postérieure 16A₁, 16B₁ de la tige, qui, par exemple, présente, en coupe transversale, un profil en creux et en bosses complémentaire de celui de la paroi du logement. En configuration d'implantation, c'est-à-dire dans la configuration du sous-ensemble 14B aux figures 3 et 4, chaque tête est totalement immobilisée par rapport à la tige correspondante. Cependant, pour faciliter l'entraînement des tiges en rotation autour de leur axe 16A₂, 16B₂ afin de les ancrer dans les pédicules vertébraux au cours de l'intervention chirurgicale d'implantation du dispositif, les tiges sont équipées, de manière amovible, d'une autre tête que la tête 18A, 18B, cette autre tête, non représentée, étant destinée à coopérer avec un outil approprié d'entraînement en rotation des tiges.

Sur son côté postérieur, chaque tête 18A, 18B est rigidement munie d'un pion 20A, 20B qui s'étend en saillie vers l'arrière depuis le reste de la face postérieure 18A₂, 18B₂ de la tête. Ce pion est dimensionné pour être reçu dans un orifice oblong 22A, 22B traversant, suivant une direction globalement antéropostérieure, la barre 12. Les orifices 22A, 22B ont leur plus grande dimension parallèle à la longueur de la barre 12. Plus précisément, le pion présente un diamètre extérieur sensiblement égal à la largeur de l'orifice oblong et inférieur à la longueur de cet orifice, notée L à la figure 3.

Comme représenté à la figure 4, la barre 12 présente, en coupe transversale, un profil en forme globale de C dont l'évidement, dirigé vers l'avant, reçoit le côté postérieur des têtes 18A et 18B. La face antérieure concave et sensiblement semi-cylindrique 12₁ de la barre est complémentaire de la face postérieure 18A₂, 18B₂, des têtes, excepté au niveau des pions 20A et 20B reçus à l'intérieur des orifices oblongs 22A, 22B. De la sorte, chaque tête est à même de coulisser le long de la barre 12, par contact glissant des faces 12₁ et 18A₂, ou 18B₂, et en étant guidée par la coopération du pion et de l'orifice oblong. Autrement dit, au niveau de chaque orifice 22A, 22B la barre 12 forme un rail de coulissement pour les têtes 18A et 18B, avec une course relative maximale de longueur L.

Pour éviter que, en service, la barre 12 ne puisse se désengager des pions 20A et 20B du côté postérieur au dispositif, chaque sous-ensemble 14A, 14B comporte une vis d'assemblage 24A, 24B dont la tige 24A₁, 24B₁ est introduite longitudinalement, depuis l'arrière du dispositif, à l'intérieur d'un trou traversant 18A₃, 18B₃ de la tête, centré sur le pion 20A, 20B et débouchant dans le logement 18A₁, 18A₂. La tête 24A₂, 24B₂ de la vis forme une butée vers l'arrière pour la barre, avec interposition d'une coiffe d'assemblage 26A, 26B à même de coulisser le long de la face postérieure convexe 12₂ de la barre 12.

Avantageusement, la tige 24A₁, 24B₁ est prévue suffisamment longue pour être vissée à l'intérieur d'un orifice longitudinal complémentaire 16A₃, 16B₃ creusé vers l'avant depuis l'extrémité postérieure 16A₁, 16B₁ de chaque tige 16A, 16B. De la sorte, dans la configuration d'implantation du dispositif, la vis 24A, 24B assure l'immobilisation axiale entre la tige 16A, 16B et la tête correspondante 18A, 18B.

Dans sa configuration d'implantation, chaque sous-ensemble 14A, 14B est ainsi relié à la barre 12 de façon coulissante avec une course maximale L. Vue latéralement comme aux figures 1 et 3, la trajectoire de coulissement le long du rachis entre chaque sous-ensemble et la barre n'est pas rectiligne mais est arquée, avec un centre de courbure situé à l'avant de la barre. Pour ce faire, la barre 12 est incurvée suivant sa longueur, en étant bombée vers l'arrière. Dans l'exemple représenté aux figures 1 à 4, la barre 12 présente un profil latéral en arc de cercle, centré en un point référencé O. La courbure de la barre 12 est prévue pour que, en configuration d'implantation du dispositif, ce centre O soit situé à l'intérieur de l'espace intervertébral séparant les corps osseux des vertèbres 1A et 1B, c'est-à-dire l'espace contenant le disque 2, en particulier dans la région centrale de cet espace. De la sorte, lorsque le dispositif de stabilisation est implanté au niveau des vertèbres 1A et 1B, les mouvements relatifs entre ces vertèbres sont, au moins en grande partie, imposés par la cinématique coulissante guidée des sous-ensembles 14A, 14B, 14A' et 14B' vis-à-vis des barres de liaison 12 et 12', les trajectoires incurvées de guidage relatif entre ces ensembles et ces barres étant respectivement référencées 28A, 28B, 28A' et 28B'. Autrement dit, chacune des trajectoires 28A, 28B, 28A' et 28B' s'étend dans un plan sensiblement parallèle au plan sagittal P et, projetée dans ce dernier, elle présente une concavité tournée vers le rachis.

De par leur rigidité structurelle, les barres de liaison 12 et 12' forment chacune un rail de guidage des sous-ensembles 14A, 14B, 14A' ou 14B' et garantissent que les centres de courbure de ces trajectoires correspondent aux centres de courbure des rails qu'elles forment, ce qui revient à dire que, sur la figure 1, les trajectoires 28A et 28B sont centrées sur le point ○. Par conséquent, ces trajectoires sont centrées sur l'espace discal intervertébral, ce qui induit un comportement dynamique proche du comportement anatomique normal de deux vertèbres adjacentes. Autrement dit, l'espace discal n'est pas réduit et le disque 2 conserve une mobilité globalement centrée sur le point 0.

En pratique, en fonction notamment des tolérances de fabrication et d'assemblage du dispositif, ainsi qu'en raison de jeux fonctionnels inhérents à cet assemblage, les trajectoires relatives entre chaque sous-ensemble et sa barre associée ne sont pas, sur toute leur course, nécessairement centrées rigoureusement en un point unique, mais plutôt en une zone regroupant l'ensemble des centres instantanés de rotation entre chaque sous-ensemble et sa barre sur la course relative maximale L. En variante non représentée, dans certains cas, le profil incurvé des barres peut d'ailleurs être prévu pour imposer, sur la course relative maximale L, plusieurs centres instantanés de rotation successifs.

Pour garantir un comportement dynamique homogène entre les vertèbres 1A et 1B sur toute la course des trajectoires 28A, 28B, 28A' et 28B', les barres de liaison 12 et 12' sont implantées de manière sensiblement parallèle l'une à l'autre, suivant une direction globalement verticale par rapport au rachis d'un patient se tenant debout.

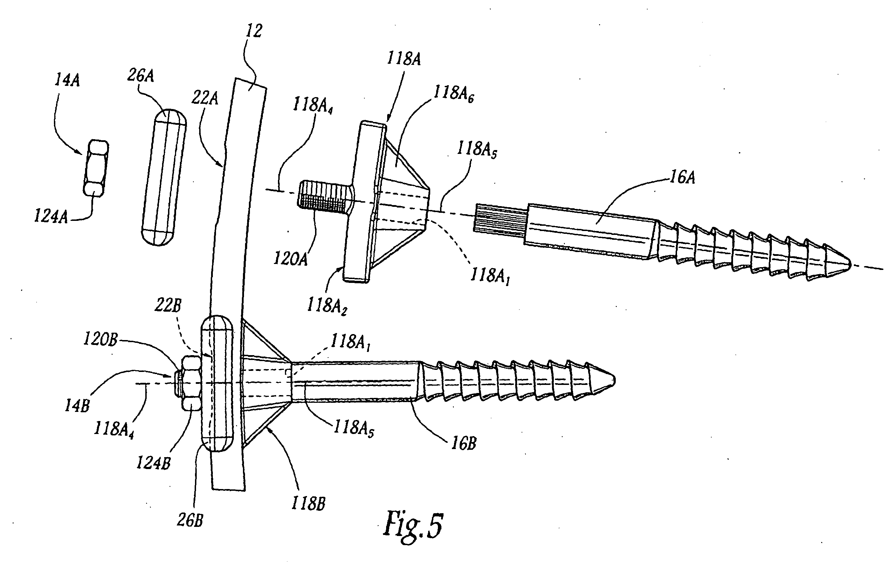

Sur les figures 5, 6, 7-8 et 9-10 sont respectivement représentées quatre variantes différentes du dispositif de stabilisation des figures 1 à 4. Par convention, les éléments identiques entre ces variantes et le dispositif des figures 1 à 4 portent les mêmes références que celles introduites ci-avant.

Le dispositif de la figure 5 se distingue de celui des figures 1 à 4 par les têtes de chacun de ses ensembles vertébraux. Au lieu du pion 20A, 20B à l'intérieur duquel est introduite une vis d'assemblage, le pion 120A, 120B de chaque tête 118A, 118B est plein et s'étend vers l'arrière, depuis la face postérieure 118A₂, de la tête, sur une longueur suffisante pour que, autour de son extrémité postérieure un écrou 124A, 124B puisse être vissé, la face extérieure du pion étant filetée à cet effet. En configuration d'implantation, cet écrou maintient le pion en travers de l'orifice oblong correspondant 22A, 22B de la barre de liaison 12, avec la même liberté de coulissement relatif que pour le dispositif des figures 1 à 4.

En raison du remplacement de la vis d'assemblage 24A, 24B par l'écrou 124A, 124B, une goupille, non représentée, ou tout autre moyen mécanique approprié est utilisé pour immobiliser axialement la tête 118A, 118B par rapport à sa tige d'ancrage 16A, 16B. L'intérêt de cette variante réside dans la possibilité de prévoir, à disposition du chirurgien, un jeu de plusieurs têtes 118A, 118B dont les axes principaux respectifs 118A₄, 118B₄, c'est-à-dire les axes longitudinaux respectifs des pions correspondant 120A, 120B, sont inclinés avec des angles respectifs différents par rapport à l'axe longitudinal 118A₅, 11BB₅ du logement 118A₁, 118B₁ de solidarisation à la tige 16A, 16B. De la sorte, une fois que le chirurgien a ancré la tige 16A, 16B, il choisit l'une des têtes parmi le jeu à sa disposition et ajuste ainsi l'orientation longitudinale du pion 120A, 120B du dispositif implanté par rapport à la tige d'ancrage. Cet ajustement permet, notamment, de rendre sensiblement perpendiculaire l'axe 118A₄, 118B₄ du pion de la tête implantée avec la direction tangentielle à la barre 12 au niveau de l'orifice 22A, 22B de réception de ce pion, ce qui facilite le mouvement de coulissement relatif entre chaque sous-ensemble vertébral et la barre.

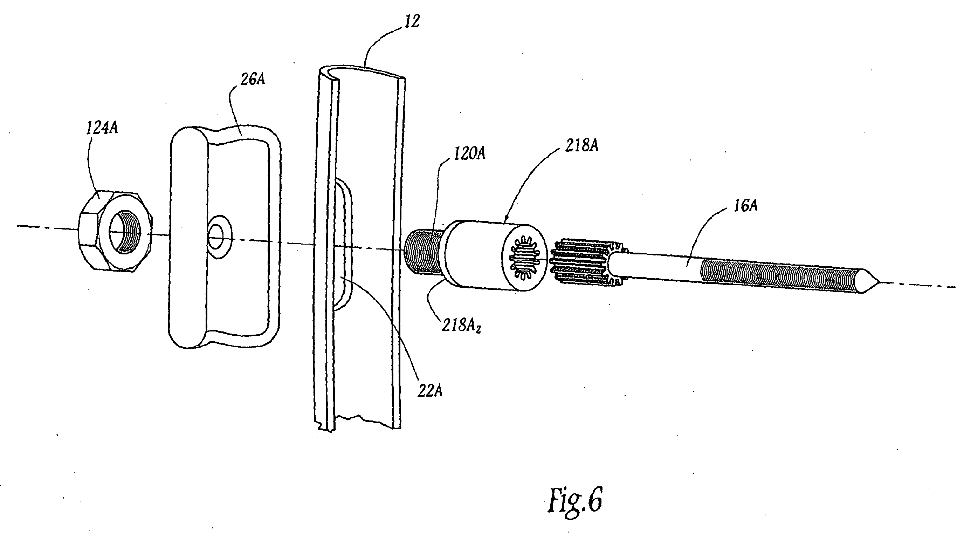

La variante de la figure 6 se distingue de celle de la figure 5 par la forme de ses têtes, dont seule la tête 218A est visible sur la figure 6, ce qui illustre la multiplicité de géométries des têtes envisageable pour le dispositif selon l'invention. Cette tête 218A est cylindrique à section externe circulaire et particulièrement compacte par rapport à la tête 118A de la figure 5, tandis que la tête 118A se révèle, à l'usage, plus résistante que la tête 218A, en raison, d'une part, de sa face postérieure 118A₂ plus étendue en longueur que la face postérieure 218A₂ de la tête 218A et, d'autre part, de la présence de renforts supérieur et inférieur 118A₆. Des renforts analogues 18A₆, 18B₆ sont d'ailleurs également présents dans le dispositif des figures 1 à 4.

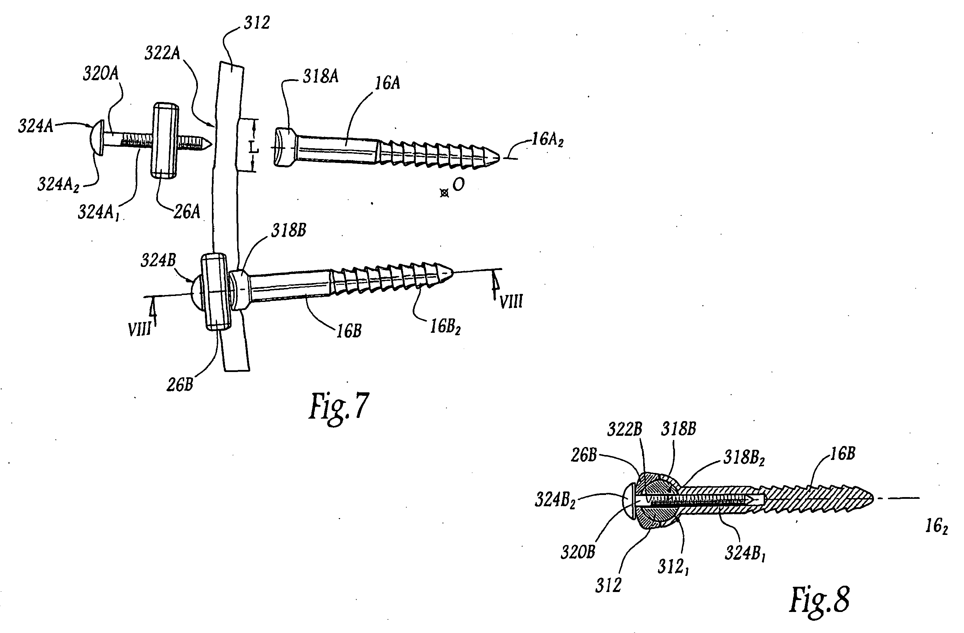

La variante de réalisation du dispositif des figures 7 et 8 se distingue du dispositif des figures 1 à 4 par le contour de la section transversale des barres de liaison reliant les sous-ensembles vertébraux. Comme représenté aux figures 7 et 8, chaque barre ou rail de liaison 312 présente ainsi une section transversale de forme sensiblement circulaire. Chaque barre forme globalement une tige arquée, centrée en un point analogue au point O pour les barres 12 et 12' du dispositif des figures 1 à 4. La section ronde de la barre 312 induit des aménagements spécifiques en ce qui concernent les composants de chaque sous-ensemble vertébral reliés de façon coulissante à cette barre. Plus précisément, dans l'exemple des figures 7 et 8, chaque tige d'ancrage osseux 16A, 16B est, à son extrémité postérieure, venue de matière avec une tête 318A, 318B en forme de demi-cylindre à base circulaire et d'axe longitudinal à la fois sensiblement perpendiculaire à l'axe 16A₂, 16B₂ de la tige et sensiblement parallèle à la barre 312. La face postérieure concave 318A₂ de chacune de ces têtes est complémentaire de la face antérieure 312₁ de la barre 312 et forme avec cette dernière un contact glissant.

Pour garantir le guidage de ce coulissement incurvé, ainsi que pour limiter la course maximale de ce coulissement, la barre 312 est traversée, suivant une direction globalement antéro-postérieure, par deux orifices distincts 322A, 322B, répartis suivant la longueur de la barre. En coupe transversale, chacun de ces orifices présente une section oblongue, de longueur L, dont la plus grande dimension est parallèle à la longueur de la barre 312. En outre, chaque sous-ensemble vertébral comporte une vis d'assemblage 324A, 324B dont la partie d'extrémité postérieure 320A, 320B de la tige forme un pion de coulissement introduit longitudinalement dans l'orifice correspondant 422A, 422B, de manière analogue au pion du dispositif des figures 1 à 4.

En outre, de manière sensiblement analogue aux vis d'assemblage 24A, 24B du dispositif des figures 1 à 4, chaque vis 324A, 324B comporte, d'une part, une partie distale de tige 324A₁, 324B₁ vissée à l'intérieur de la tige d'ancrage 16A, 16B et, d'autre part, une tête 324A₂, 324B₂ de maintien d'une coiffe 26A, 26B montée à coulissement sur la face postérieure 312₂ de la barre ou rail 312.

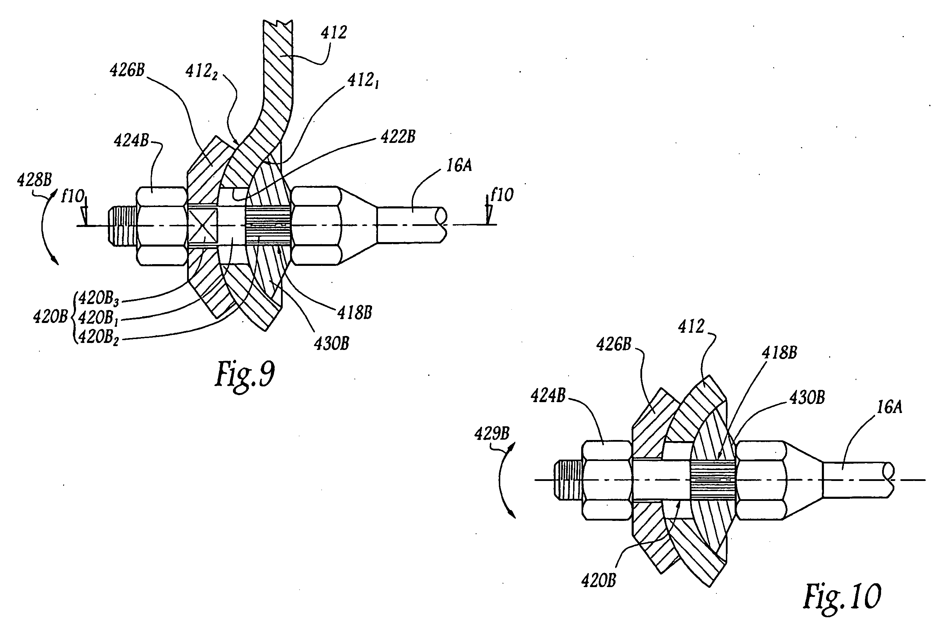

La variante de réalisation des figures 9 et 10 se distingue des dispositifs des figures 1 à 8 par une plus grande liberté de mouvements relatifs entre chaque sous-ensemble vertébral et sa barre de guidage associée. En effet, plutôt que de prévoir des orifices oblongs pour recevoir la tête de chaque sous-ensemble, la barre 412 représentée, en partie seulement, aux figures 9 et 10 est traversée de part en part, suivant une direction antéropostérieure, par un orifice 422B de section sensiblement circulaire, qui reçoit la tête 418B du sous-ensemble visible aux figures, un autre orifice à section sensiblement circulaire étant prévu dans la partie d'extrémité de la barre opposée à celle qui est représentée. Par commodité, seul le sous-ensemble visible aux figures 9 et 10 sera détaillé plus avant, étant entendu que, comme pour les dispositifs décrits ci-dessus, les autres sous-ensembles du dispositif présentent des aménagements analogues.

La tête 418B est prévue, dans sa partie d'extrémité antérieure, pour être solidarisée à la tige d'ancrage osseux 16A, suivant les mêmes aménagements que décrits plus haut pour les dispositifs des figures 1 à 8. Dans sa partie d'extrémité postérieure, la tige 418B forme un pion 420B dont la partie courante 420B, sensiblement cylindrique présente un diamètre largement inférieur à celui de l'orifice 422B dans lequel cette partie 420B₁ est logée dans la configuration d'implantation du dispositif. L'extrémité antérieure 420B₂ du pion est solidarisée à une rondelle antérieure 430B, par exemple par coopération de profils en creux et en bosse conjugués, portés par le pion et la rondelle. De même, l'extrémité postérieure 420B₃ du pion 420B est solidaire d'une rondelle postérieure 426B. Cette rondelle 426B fait office de coiffe d'assemblage du dispositif, un écrou de maintien 424B, fonctionnellement analogue à l'écrou 124B du dispositif des figures 5 et 6, étant prévu à l'extrémité postérieure du pion.

Lorsque le dispositif des figures 9 et 10 est implanté sur le rachis, le sous-ensemble représenté aux figures est à même de se déplacer par rapport à la barre 412 en raison de l'écartement périphérique entre le pion 420B et la paroi de l'orifice 422B. Si on regarde le dispositif latéralement au rachis, comme à la figure 9, un mouvement de coulissement, globalement parallèle à la direction longitudinale du rachis, est permis entre le sous-ensemble et la barre, ce mouvement étant analogue à celui correspondant à la trajectoire 28B aux figures 1 et 3. Autrement dit, la projection de la trajectoire de déplacement sur le plan sagittal P du rachis, référencée 428B à la figure 9 et qui constitue la composante sagittale de cette trajectoire, est incurvée en étant bombée vers l'arrière. En plus de cette composante sagittale, la trajectoire présente une composante transversale non nulle, correspondant à la projection de la trajectoire dans un plan horizontal par rapport au rachis d'un patient se tenant debout. On notera que, pour les modes de réalisation des figures 1 à 8, les trajectoires de guidage 28A, 28B, 28A', 28B' ne présentent pas de composante sagittale, à des jeux fonctionnels près. Grâce à cette composante transversale, référencée 429B à la figure 10, le dispositif des figures 9 et 10 présente un débattement interne transversal à la direction longitudinale du rachis, assurant un plus grand confort au patient lors de mouvements combinés de torsion et de flexion-extension du rachis.

On comprend qu'à cet effet, les rondelles 426B et 430B sont respectivement adaptées pour glisser contre les faces antérieure 412₁ et postérieure 412₂ de la barre 412, de manière à guider de manière incurvée les mouvements de débattement entre la tête 418B et la barre, sans les gêner.

En pratique, la surface postérieure 430B₁ de la rondelle 430B et la surface coopérante délimitée par la face antérieure 412₁ de la barre 412 correspondent sensiblement à une même portion de sphère dont la concavité est tournée vers le rachis. Il en est avantageusement de même pour la surface antérieure 426B₁ de la rondelle 426B et la surface coopérante délimitée par la face postérieure 412₂.

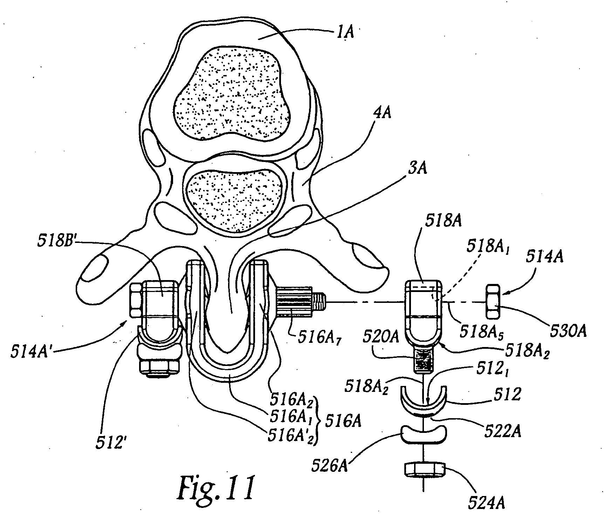

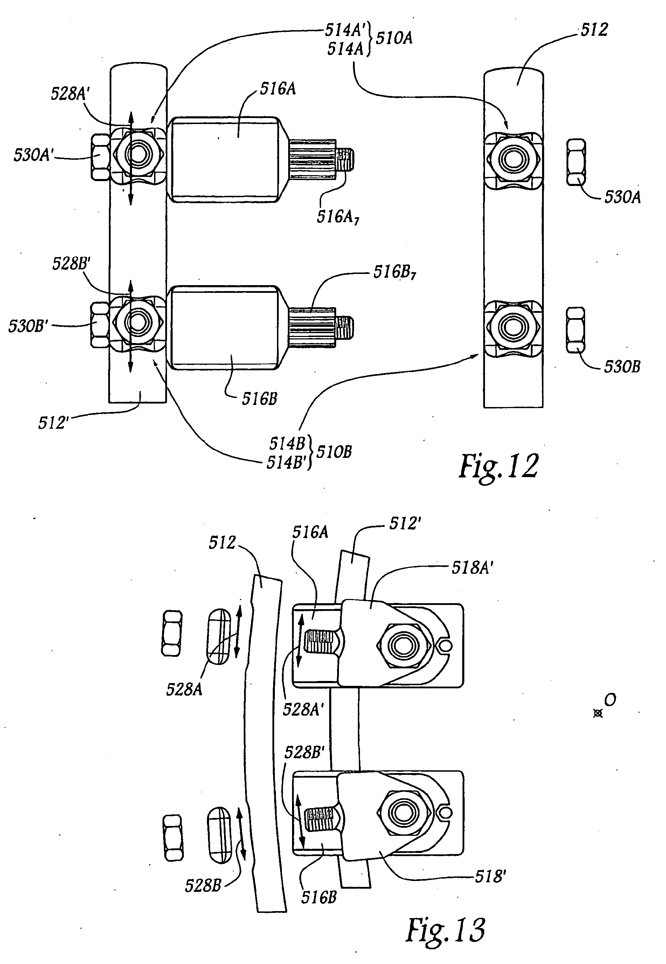

Sur les figures 11 à 13 est représenté un second mode de réalisation du dispositif de stabilisation du rachis. Comme pour les dispositifs des figures 1 à 8, le dispositif des figures 11 à 13 comporte essentiellement un ensemble vertébral 510A destiné à être implanté au niveau de la vertèbre 1A, un ensemble vertébral 510B destiné à être implanté au niveau de la vertèbre 1B et deux barres ou rails 512, 512' reliant l'un à l'autre ces deux ensembles. Chaque ensemble vertébral 510A, 510B comprend un sous-ensemble vertébral droit 514A, 514B et un sous-ensemble vertébral gauche 514A', 514B', la barre 512 reliant les sous-ensemble droit tandis que la barre 512' relie les sous-ensembles gauches.

Ce second mode de réalisation se distingue essentiellement du dispositif de la figure 5 par la zone de fixation osseuse sur les vertèbres 1A et 1B. Plutôt que de prévoir une fixation pédiculaire, chaque ensemble 510A, 510B comporte une pince 516A, 516B destinée à enserrer, depuis l'arrière, l'apophyse épineuse 3A, 3B de chaque vertèbre, comme représenté à la figure 11. Chaque pince est commune aux deux sous-ensembles vertébraux de l'ensemble considéré, ce qui réduit le nombre de composants du dispositif par rapport à ceux des figures 1 à 10.

Chaque pince 516A, 516B présente, en coupe transversale, c'est-à-dire dans un plan de coupe sensiblement vertical lorsque cette pince est en prise sur son apophyse 3A, 3B, un profil globalement en forme de U, dont le fond 516A₁, est dirigé vers l'arrière tandis que les deux ailes 516A₂ droite et 516A₂' gauche sont disposées latéralement de part et d'autre de l'apophyse. Pour améliorer la tenue mécanique de la fixation de chaque pince, les faces en regard des branches présentent un relief en bosses et en creux, adapté pour agripper la matière osseuse apophysaire.

Chaque ensemble vertébral 510A, 510B comporte également des composants respectivement associés au côté droit et gauche de l'apophyse 3A, 3B, seuls les composants du sous-ensemble 514A étant détaillés ci-après, étant entendu que les autres sous-ensembles 514B, 514A' et 514B' comportent des composants analogues, avec les conventions de référence expliquées plus haut.

Le sous-ensemble 514A comporte une tête massive 518A présentant une face postérieure 518A₂ sensiblement semi-cylindrique convexe et prévue pour coulisser le long de la barre 512 à section en C, contre sa face antérieure 512₁. Cette tête présente, sur son côté postérieur, un pion 520A similaire au pion 120A du dispositif de la figure 5 et reçu dans un orifice oblong 522A traversant la barre 512 de part en part et dont le plus grand axe est parallèle à la longueur de la barre 512. Ce pion est associé à un écrou 524A et à une coiffe d'assemblage 526A analogues à l'écrou 124A et à la coiffe 26A. En outre, la tête 518A est fixement solidarisée à l'aile correspondante 516A₂ de la pince 516A par assemblage de cette tête à un cylindre de support correspondant 516A₇ venu de matière avec cette aile. Plus précisément, la tête présente un orifice traversant 518A₁ adapté pour être enfilé autour du cylindre de support 516A₇, un écrou additionnel 530A étant rapporté du côté de la tête opposé à l'aile, en étant vissé sur une extrémité filetée correspondante du cylindre de support, pour immobiliser la tête suivant l'axe 518A₅ de ce logement.

Avantageusement, la face extérieure du cylindre de support 516A₇ et la paroi du logement 518A₁ sont adaptées pour permettre d'ajuster la position angulaire de la tête par rapport à ce cylindre, autour de l'axe 518A₅, avant que l'écrou 530A ne soit fermement serré. De la sorte, alors que le dispositif est en cours d'implantation, le chirurgien peut ajuster la position de la tête 518A par rapport à la pince 516A en entraînant cette tête en rotation autour de l'axe 518A₅, notamment en vue de rendre l'axe longitudinal 518A₄ du pion 520A sensiblement perpendiculaire à la direction tangentielle à la barre 512 au niveau de son orifice de réception 522A. Autrement dit, le dispositif des figures 11 à 13 permet, avant que le dispositif ne soit figé dans sa configuration d'implantation, d'ajuster la direction longitudinale du pion de coulissement de chaque tête par rapport aux composants du dispositif solidarisés fixement aux vertèbres.

En fonctionnement, le dispositif des figures 11 à 13 se comporte de manière identique à celui des figures 1 à 4 puisque chaque barre 512, 512' est incurvée de manière analogue aux barres 12 et 12', en étant disposée, parallèlement l'une à l'autre, de part et d'autre des apophyses 3A et 3B. Les trajectoires de guidage incurvées, entre chaque sous-ensemble 514A, 514B, 514A', 514B' et les barres 512, 512', respectivement référencées 528A, 528B, 528A', 528B', sont centrées en un point O.

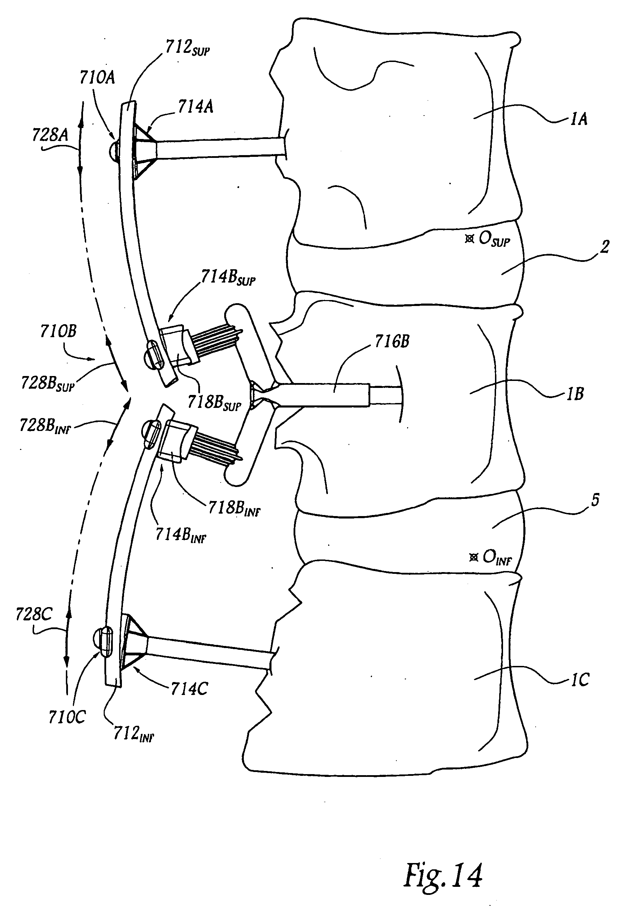

Sur la figure 14 est représentée une variante du dispositif de stabilisation intervertébrale, destiné à être implanté au niveau de trois vertèbres adjacentes 1A, 1B et 1C séparées deux à deux par des disques 2 et 5. Pour des raisons de visibilité, seuls les corps vertébraux de ces vertèbres sont représentés, de manière schématique.

Ce dispositif correspond, en quelque sorte, à la juxtaposition de deux dispositifs des figures 1 à 4. Plus précisément, ce dispositif comprend trois ensembles vertébraux 710A, 710B et 710C ancrés dans les pédicules des vertèbres 1A, 1B et 1C. Une paire de barres incurvées relie les ensembles 710A et 710B, de la même façon que les barres 12 et 12' relient les ensembles 10A et 10B aux figures 1 à 4, tandis qu'une autre paire de barres relie les ensembles 710B et 710C, également de la même façon que les barres 12 et 12' relient les ensembles 10A et 10B aux figures 1 à 4. Sur la figure 14 illustrant le côté droit du dispositif, seules sont visibles une barre 712_{SUP} reliant les ensembles 710A et 710B et une barre 712_{INF} reliant les ensembles 710B et 710C. Les ensembles 710A et 710B sont reliés de façon coulissante à la barre 712_{SUP}, suivant des trajectoires incurvées de guidage relatif, référencées 728A, 728B_{SUP} et centrées en un point O_{SUP}, tandis que l'ensemble 710D et 710C sont reliés à la barre 712_{INF} de façon coulissante suivant des trajectoires de guidage incurvées 728B_{INF} et 728C centrées en un point O_{INF}. Le centre O_{SUP} est situé dans l'espace intervertébral occupé par le disque 2, tandis que le centre O_{INF} est situé dans l'espace intervertébral occupé par le disque 5.

Par commodité, seul le côté droit de ce dispositif, visible à la figure 14, est détaillé ci-après, étant entendu que des aménagements analogues sont prévus du côté gauche du dispositif, de manière sensiblement symétrique à un plan sagittal passant par les apophyses épineuses des vertèbres. Ainsi, le sous-ensemble droit 710B comporte à la fois un sous-ensemble supérieur 714B_{SUP} et un sous-ensemble inférieur 714B_{INF}, tous deux portés par une même tige d'ancrage pédiculaire 716B. Chacun de ces sous-ensembles comportent une tête 718A_{SUP}, 718A_{INF} sensiblement analogue à la tête de chaque sous-ensemble du dispositif de la figure 6. Les sous-ensembles droits 714A et 714C sont, quant à eux, analogues aux sous-ensembles 14A et 14B des figures 1 à 4.

En service, le dispositif de la figure 14 assure des cinématiques propres à chaque paire de vertèbres 1A/1B et 1B/1C, respectivement analogues à la cinématique détaillée pour les vertèbres 1A/1B des figures 1 à 4.

Divers aménagements et variantes aux dispositifs de stabilisation décrits ci-dessus sont en outre envisageables :
- les formes des sous-ensembles vertébraux des dispositifs pour trois vertèbres ne sont pas limitées à celles représentées à la figure 14, mais ces sous-ensembles peuvent prendre indifféremment les formes des sous-ensembles envisagés aux figures 1 à 13 ;
- les barres ou rails de liaison coulissante entre les sous-ensembles vertèbraux ne sont pas nécessairement prévus pour être implantés en regard du côté postérieur des vertèbres ; des barres ou rails décalés latéralement à droite et à gauche des vertèbres, ou bien agencés du côté antérieur des vertèbres sont envisageables ; dans le cas de rails prévus du côté antérieur, ces rails sont de préférence portés par une pièce commune, notamment une plaque, plus facile à mettre en place que deux barres indépendantes l'une de l'autre ;
- dans tous les exemples de réalisation envisagés sur les figures, les barres de liaison présentent une courbure continue sur toute leur longueur, de sorte que les trajectoires de coulissement relatif entre chaque sous-ensemble vertébral et cette barre sont centrées en un point unique, ou tout au moins en une zone unique ; on peut envisager de conformer chaque barre avec des courbures différentes au niveau de ses orifices oblongs de guidage en coulissement de chaque sous-ensemble, de sorte que, pour une barre donnée, les deux trajectoires coulissantes associées respectivement à chaque sous-ensemble vertébral sont alors centrées en deux points, ou tout au moins en deux zones, distinctes l'un de l'autre, ces deux points étant cependant situés tous les deux à l'intérieur de l'espace inter-osseux délimité entre les deux vertèbres auxquelles les ensembles vertébraux sont fixés ;
- la partie médiane de chaque barre ou rail, reliant les deux parties extrêmes du rail le long desquelles coulissent les sous-ensembles vertébraux, peut présenter une structure rectiligne ou autre, puisqu'elle n'a pas d'incidence sur la courbure des trajectoires de guidage relatif ; et/ou
- les vertèbres peuvent être appareillées sur un seul côté; dans ce cas, chaque ensemble vertébral ne comprend qu'un seul sous-ensemble.

## Revendications

1. Dispositif de stabilisation dynamique du rachis destiné à reproduire une liaison intervertébrale articulaire, comportant au moins deux ensembles vertébraux (10A, 10B ; 510A, 510B ; 710A-710C) adaptés pour être respectivement fixés chacun à l'os d'une vertèbre parmi au moins deux vertèbres différentes (1A, 1B ; 1A, 1C ; 1A, 1B, 1C) du rachis, ledit dispositif comportant en outre des moyens rigides (12, 12' ; 312 ; 412 ; 512, 512' ; 712_{SUP}, 712_{INF}) de liaison entre les deux ensembles vertébraux (10A, 10B ; 510A, 510B) ou entre deux desdits ensembles vertébraux (710A-710C), **caractérisé en ce que** lesdits moyens rigides et lesdits ensembles vertébraux sont adaptés pour, lorsque le dispositif est en configuration d'implantation, être reliés les uns aux autres de façon coulissante suivant une trajectoire de guidage relatif (28A, 28B, 28A', 28B' ; 428B, 429B ; 528A, 528B, 528A', 528B' ; 728A, 728B_{SUP}, 728B_{INF}, 728C) qui, projetée dans le plan sagittal (P) du rachis, est incurvée le long du rachis, en présentant une concavité tournée vers le rachis et en étant centrée en un point (O ; O_{SUP}, O_{INF}) situé dans l'espace inter-osseux délimité entre les deux vertèbres auxquelles les deux ensembles sont fixés.

2. Dispositif suivant la revendication 1, **caractérisé en ce que** les ensembles vertébraux (10A, 10B ; 510A, 510B) sont respectivement adaptés pour être fixés sur deux vertèbres adjacentes (1A, 1B), et **en ce que** la projection, dans le plan sagittal (P) du rachis, de la trajectoire de guidage relatif (28A, 28B, 28A', 28B' ; 428B, 429B ; 528A, 528B, 528A', 528B'), entre les moyens de liaison (12, 12' ; 412 ; 512, 512') et chacun des deux ensembles vertébraux associés, est centrée en un point (O) situé dans l'espace discal séparant les deux vertèbres auxquelles les deux ensembles sont fixés.

3. Dispositif suivant l'une quelconque des revendications précédentes, **caractérisé en ce que** chaque ensemble vertébral (10A, 10B ; 510A, 510B ; 710A-710C) comprend deux sous-ensembles (14A, 14A', 14B, 14B' ; 514A, 514A', 514B, 514B' ; 714A, 714B_{SUP}, 714B_{INF}, 714C) aptes à être fixés sur une même vertèbre (1A, 1B), de part et d'autre de son apophyse épineuse (3A, 3B).

4. Dispositif suivant l'une des revendications précédentes, **caractérisé en ce que** les moyens de liaison des deux ensembles comportent deux rails rigides incurvés (12, 12' ; 312 ; 512, 512' ; 712_{SUP}, 712_{INF}) de guidage des ensembles vertébraux, rails sensiblement parallèles l'un à l'autre et le long desquels, respectivement, des parties latérales opposées (14A, 14B, 14A', 14B' ; 514A, 514B, 514A', 514B' ; 714A, 714B, 714C) de chaque ensemble vertébral (10A, 10B ; 510A, 510B ; 710A, 710B, 710C) sont adaptées pour coulisser suivant ladite trajectoire (28A, 28B, 28A', 28B' ; 428B, 429B ; 528A, 528B, 528A', 528B' ; 728A, 728B_{SUP}, 728B_{INF}, 728C) lorsque le dispositif est en configuration d'implantation.

5. Dispositif suivant la revendication 4, **caractérisé en ce que** les deux rails (12, 12' ; 312 ; 412 ; 512, 512' ; 712_{SUP}, 712_{INF}) sont adaptés pour s'étendre le long et de part et d'autre des apophyses épineuses (3A, 3B ; 3A, 3B, 3C) des vertèbres (1A, 1B ; 1A, 1B, 1C).

6. Dispositif suivant la revendication 4, **caractérisé en ce que** les deux rails sont portés par une même pièce adaptée pour s'étendre, suivant la direction longitudinale des rails, le long du côté antérieur des vertèbres.

7. Dispositif suivant l'une quelconque des revendications 5 à 7, **caractérisé en ce que** chaque partie latérale (14A, 14B, 14A', 14B' ; 514A, 514B, 514A', 514B' ; 714A, 714B, 714C) de chaque ensemble vertébral (10A, 10B ; 510A, 510B ; 710A, 710B, 710C) comporte une tête (18A, 18B, 18A', 18B' ; 118A, 118B ; 218A ; 318A, 318B ; 418B ; 518A, 518B, 518A', 518B') de coulissement le long du rail correspondant (12, 12' ; 312 ; 412 ; 512, 512' ; 712_{SUP}, 712_{INF}), cette tête étant munie d'un pion (20A, 20B, 20A', 20B' ; 120A, 120B ; 320A, 320B ; 420B ; 520A, 520B, 520A', 520B') reçu dans un orifice de guidage (22A, 22B ; 322A, 322B ; 422B, 522A, 522B) délimite par le rail.

8. Dispositif suivant l'une quelconque des revendications 5 à 7, **caractérisé en ce que** chaque partie latérale (14A, 14B, 14A', 14B' ; 514A, 514B, 514A', 514B') de chaque ensemble vertébral (10A, 10B, 510A, 510B) comporte une tige d'ancrage pédiculaire (16A, 16B, 16A', 16B') ou une pince d'accrochage apophysaire (516A, 516B, 516A', 516B').

9. Dispositif suivant les revendications 7 et 8 prises ensemble, **caractérisé en ce que** la direction longitudinale (118A₄, 518A₄) du pion (120A, 520A) de chaque tête (118A, 518A) est ajustable par rapport à la tige (16A) ou à la pince (516A) avant de passer le dispositif en configuration d'assemblage.

10. Dispositif suivant les revendications 7 et 8 prises ensemble, **caractérisé en ce que** chaque tête (18A, 118A, 518A) est amovible par rapport à la tige (16A) ou à la pince (516A) avant de passer le dispositif en configuration d'assemblage.

11. Dispositif suivant l'une quelconque des revendications 7 à 10, **caractérisé en ce que** ledit orifice de guidage (22A, 22B ; 322A, 322B ; 522A, 522B) a une forme oblongue, dont la plus grande dimension s'étend suivant la longueur du rail correspondant (12, 12' ; 312 ; 512, 512' ; 712_{SUP}, 722_{INF}).

12. Dispositif suivant l'une quelconque des revendications 1 à 10, **caractérisé en ce que,** projetée dans un plan horizontal au rachis, la trajectoire (428B, 429B) de guidage relatif, entre les moyens de liaison (412) et chacun des ensembles vertébraux associés, présente une composante (429B) non nulle.

13. Dispositif suivant la revendication 12, **caractérisé en ce que** les moyens de liaison (412) et chacun des ensembles vertébraux associés sont adaptés pour glisseur les uns contre les autres au niveau d'au moins deux surfaces respectives (430B₁, 412₁, 426B₁, 412₂) de guidage relatif, qui correspondent sensiblement à une même portion de sphère de concavité tournée vers le rachis.

14. Dispositif suivant l'une quelconque des revendications précédentes, **caractérisé en ce que** les trajectoires de guidage relatif, entre les moyens de liaison et les deux ensembles vertébraux associés, sont respectivement centrées en des points distincts.

## Claims

1. Device for the dynamic stabilisation of the spine, said device being intended to reproduce an articular intervertebral link and comprising at least two vertebral assemblies (10A, 10B; 510A, 510B; 710A-710C) which are adapted to each be fixed, respectively, to the bone of one of at least two different vertebrae (1A, 1B; 1A, 1C; 1A, 1B, 1C) of the spine, the said device also comprising rigid means (12, 12'; 312; 412; 512, 512'; 712_{SUP}, 712_{INF}) providing a link between the two vertebral assemblies (10A, 10B; 510A, 510B) or between two of the said vertebral assemblies (710A-710C), **characterised in that** the said rigid means and the said vertebral assemblies are adapted to be connected to one another, when the device is in the implantation configuration, so as to slide along a relative guidance trajectory (28A, 28B, 28A', 28B'; 428B, 429B; 528A, 528B, 528A', 528B'; 728A, 728B_{SUP}, 728B_{INF}, 728C) which, projected into the sagittal plane (P) of the spine, is incurved along the spine while exhibiting a concavity which is turned towards the spine and being centred at a point (O; O_{SUP}, O_{INF}) which is situated in the inter-osseous space delimited between the two vertebrae to which the two assemblies are fixed.

2. Device according to claim 1, **characterised in that** the vertebral assemblies (10A, 10B; 510A, 510B) are respectively adapted to be fixed onto two adjacent vertebrae (1A, 1B), and **in that** the projection, into the sagittal plane (P) of the spine, of the relative guidance trajectory (28A, 28B, 28A', 28B'; 428B, 429B; 528A, 528B, 528A', 528B'), between the linking means (12, 12'; 412; 512, 512') and each of the two associated vertebral assemblies, is centred at a point (O) which is situated in the discal space separating the two vertebrae to which the two assemblies are fixed.

3. Device according to any of the preceding claims, **characterised in that** each vertebral assembly (10A, 10B; 510A, 510B; 710A-710C) comprises two subassemblies (14A, 14A', 14B, 14B'; 514A, 514A', 514B, 514B'; 714A, 714B_{SUP}, 7142_{INF}, 714C) which are capable of being fixed onto the same vertebra (1A, 1B) on either side of its spiny apophysis (3A, 3B).

4. Device according to one of the preceding claims, **characterised in that** the means for linking the two assemblies comprise two incurved, rigid rails (12, 12'; 312; 512, 512'; 712_{SUP}, 712_{INF}) for guiding the vertebral assemblies, which rails are substantially parallel to one another and along which, respectively, opposed lateral parts (14A, 14B, 14A', 14B'; 514A, 514B, 514A', 514B'; 714A, 714B, 714C) of each vertebral assembly (10A, 10B; 510A, 510B; 710A, 710B, 710C) are adapted to slide along the said trajectory (28A, 28B, 28A', 28B'; 428B, 429B; 528A, 528B, 528A', 528B'; 728A, 728B_{SUP}, 728B_{INF}, 728C) when the device is in the implantation configuration.

5. Device according to claim 4, **characterised in that** the two rails (12, 12'; 312; 412; 512, 512'; 712_{SUP}, 712_{INF}) are adapted to extend along, and on either side of, the spiny processes (3A, 3B; 3A, 3B, 3C) of the vertebrae (1A, 1B; 1A, 1B, 1C).

6. Device according to claim 4, **characterised in that** the two rails are carried by the same piece which is adapted to extend, in the longitudinal direction of said rails, along the anterior side of the vertebrae.

7. Device according to any of claims 5 to 7, **characterised in that** each lateral part (14A, 14B, 14A', 14B'; 514A, 514B, 514A', 514B'; 714A, 714B, 714C) of each vertebral assembly (10a, 10B; 510A, 510B; 710A, 710B, 710C) comprises a head (18A, 18B, 18A', 18B'; 118A, 118B; 218A; 318A, 318B; 418B; 518A, 518B, 518A', 518B') for sliding along the corresponding rail (12, 12'; 312; 412; 512, 512'; 712_{SUP}, 712_{INF}), the said head being provided with a stud-bolt (20A, 20B, 20A', 20B'; 120A, 120B; 320A, 320B; 420B; 520A, 520B, 520A', 520B') which is received in a guide orifice (22A, 22B; 322A, 322B; 422B; 522A, 522B) which is delimited by the rail.

8. Device according to any of claims 5 to 7, **characterised in that** each lateral part (14A, 14B, 14A', 14B'; 514A, 514B, 514A', 514B') of each vertebral assembly (10a, 10B; 510A, 510B) comprises a pedicular anchoring rod (16A, 16B, 16A', 16B') or an apophyseal coupling gripper (516A, 516B, 516A', 516B').

9. Device according to claims 7 and 8, taken together, **characterised in that** the longitudinal direction (118A₄, 518A₄) of the stud-bolt (120A, 520A) of each head (118A, 518A) is adjustable in relation to the rod (16A) or to the gripper (516A) before the device is moved into the assembly configuration.

10. Device according to claims 7 and 8, taken together, **characterised in that** each head (18A, 118A, 518A) is detachable in relation to the rod (16A) or to the gripper (516A) before the device is moved into the assembly configuration.

11. Device according to any of claims 7 to 10, **characterised in that** the said guide orifice (22A, 22B; 322A, 322B; 522A, 522B) has an oblong shape, the largest dimension of which extends along the length of the corresponding rail (12, 12'; 312; 512, 512'; 712_{SUP}, 712_{INF}).

12. Device according to any of claims 1 to 10, **characterised in that,** projected into a plane horizontal to the spine, the relative guidance trajectory (428B, 429B) between the linking means (412) and each of the associated vertebral assemblies, has a non-null component (429B).

13. Device according to claim 12, **characterised in that** the linking means (412) and each of the associated vertebral assemblies are adapted to slide against one another at at least two respective relative guidance surfaces (430B₁, 412₁, 426B₁, 412₂) which substantially correspond to the same portion of a sphere having a concavity which is turned towards the spine.

14. Device according to any of the preceding claims, **characterised in that** the relative guidance trajectories between the linking means and the two associated vertebral assemblies are respectively centred at distinct points.

## Patentansprüche

1. Vorrichtung zur dynamischen Stabilisierung der Wirbelsäule, die dazu bestimmt ist, eine gelenkartige Verbindung zwischen den Wirbeln nachzubilden, und die mindestens zwei wirbelseitige Baugruppen (10A, 10B; 510A, 510B; 710A-710C) aufweist, die jeweils dazu geeignet sind, am Knochen eines von mindestens zwei verschiedenen Wirbeln (1A, 1B; 1A, 1C; 1A, 1B, 1C) der Wirbelsäule befestigt zu werden, wobei die Vorrichtung darüber hinaus steife Mittel (12, 12'; 312; 412; 512, 512'; 712_{SUP}, 712_{INF}) aufweist, welche die Verbindung zwischen den zwei wirbelseitigen Baugruppen (10A, 10B; 510A, 510B) oder zwischen zwei der wirbelseitigen Baugruppen (710A-710C) herstellen, **dadurch gekennzeichnet, dass** die steifen Mittel und die wirbelseitigen Baugruppen derart ausgeführt sind, dass sie, nachdem die Vorrichtung implantiert wurde, derart miteinander verbunden werden können, dass sie sich entlang einer relativen Führungsbahn (28A, 28B, 28A', 28B'; 428B, 429B; 528A, 528B, 528A', 528B'; 728A, 728B_{SUP}, 728B_{INF}, 728C) gegeneinander verschieben können, wobei die Führungsbahn, wenn sie auf die Sagittalebene (P) der Wirbelsäule projiziert wird, entlang der Wirbelsäule eine Kurve beschreibt, wobei sie eine konkave Ausbuchtung zur Wirbelsäule hin aufweist und einen Mittelpunkt (0; O_{SUP}, O_{INF}) aufweist, der im Zwischenknochenraum gelegen ist, welcher durch die beiden Wirbel begrenzt ist, an denen die zwei Baugruppen befestigt sind.

2. Vorrichtung nach Anspruch 1, **dadurch gekennzeichnet, dass** die wirbelseitigen Baugruppen (10A, 10B; 510A, 510B) jeweils dazu geeignet sind, an zwei benachbarten Wirbeln (1A, 1B) befestigt zu werden, und dass die relative Führungsbahn (28A, 28B, 28A', 28B'; 428B, 429B; 528A, 528B, 528A', 528B'), wenn sie auf die Sagittalebene (P) der Wirbelsäule projiziert wird, zwischen den Verbindungsmitteln (12, 12'; 412; 512, 512') und jeder der beiden damit verbundenen wirbelseitigen Baugruppen einen Mittelpunkt (O) aufweisen, der im Bandscheibenbereich zwischen den beiden Wirbeln, an denen die Baugruppen befestigt sind, gelegen ist.

3. Vorrichtung nach einem beliebigen der vorhergehenden Ansprüche, **dadurch gekennzeichnet, dass** jede wirbelseitige Baugruppe (10A, 10B; 510A, 510B; 710A-710C) zwei Unterbaugruppen (14A, 14A', 14B, 14B'; 514A, 514A', 514B, 514B'; 714A, 714B_{SUP}, 714B_{INF}, 714C) umfasst, die dazu geeignet sind, an ein und demselben Wirbel (1A, 1B) beiderseits von dessen Dornfortsatz (3A, 3B) befestigt zu werden.

4. Vorrichtung nach einem der vorhergehenden Ansprüche, **dadurch gekennzeichnet, dass** die Mittel zur Verbindung der beiden Baugruppen zwei steife Schienen (12, 12'; 312; 512, 512'; 712_{SUP}, 712_{INF}) zur Führung der wirbelseitigen Baugruppen aufweisen, wobei die Schienen eine Kurve beschreiben und im Wesentlichen parallel zueinander verlaufen und wobei die jeweils sich gegenüberliegenden seitlichen Abschnitte (14A, 14B, 14A', 14B'; 514A, 514B, 514A', 514B'; 714A, 714B, 714C) jeder wirbelseitigen Baugruppe (10A, 10B; 510A, 510B; 710A, 710B, 710C) dazu geeignet sind, sich der Bahn (28A, 28B, 28A', 28B'; 428B, 429B; 528A, 528B, 528A', 528B'; 728A, 728B_{SUP}, 728B_{INF}, 728C) folgend entlang den Schienen zu verschieben, nachdem die Vorrichtung implantiert wurde.

5. Vorrichtung nach Anspruch 4, **dadurch gekennzeichnet, dass** die beiden Schienen (12, 12'; 312; 412; 512, 512'; 712_{SUP}, 712_{INF}) derart ausgeführt sind, dass sie sich beiderseits entlang der Dornfortsätze (3A, 3B; 3A, 3B, 3C) der Wirbel (1A, 1B; 1A, 1B, 1C) erstrecken.

6. Vorrichtung nach Anspruch 4, **dadurch gekennzeichnet, dass** die beiden Schienen von ein und demselben Bauteil getragen werden, welches derart ausgeführt ist, dass es sich, der Längsrichtung der Schienen folgend, entlang der Vorderseite der Wirbel erstreckt.

7. Vorrichtung nach einem beliebigen der vorhergehenden Ansprüche 5 bis 7, **dadurch gekennzeichnet, dass** jeder seitliche Abschnitt (14A, 14B, 14A', 14B'; 514A, 514B, 514A', 514B'; 714A, 714B, 714C) jeder wirbelseitigen Baugruppe (10A, 10B; 510A, 510B; 710A, 710B, 710C) einen Kopf (18A, 18B, 18A', 18B'; 118A, 118B; 218A; 318A, 318B; 418B; 518A, 518B, 518A', 518B') zum verschieben entlang der entsprechenden Schiene (12, 12'; 312; 412; 512, 512'; 712_{SUP}, 712_{INF}) aufweist, wobei dieser Kopf mit einem Stift (20A, 20B, 20A', 20B'; 120A, 120B; 320A, 320B; 420B; 520A, 520B, 520A', 520B') versehen ist, der von einer Führungsöffnung (22A, 22B; 322A, 322B; 422B, 522A, 522B) aufgenommen wird, die von der Schiene begrenzt wird.

8. Vorrichtung nach einem beliebigen der vorhergehenden Ansprüche 5 bis 7, **dadurch gekennzeichnet, dass** jeder seitliche Abschnitt (14A, 14B, 14A', 14B'; 514A, 514B, 514A', 514B') jeder wirbelseitigen Baugruppe (10A, 10B, 510A, 510B) eine Stange (16A, 16B, 16A', 16B') zur Verankerung im Wirbelbogenansatz oder eine Klemme (516A, 516B, 516A', 516B') zur Befestigung an einem Fortsatz aufweist.

9. Vorrichtung nach den Ansprüchen 7 und 8 in ihrer Gesamtheit, **dadurch gekennzeichnet, dass** die Längsausrichtung (118A₄, 518A₄) des Stifts (120A, 520A) jedes Kopfes (118A, 518A) in Bezug auf die Stange (16A) oder an die Klemme (516A) angepasst werden kann, bevor die Vorrichtung zusammengesetzt wird.

10. Vorrichtung nach den Ansprüchen 7 und 8 in ihrer Gesamtheit, **dadurch gekennzeichnet, dass** jeder Kopf (18A, 118A, 518A) gegenüber der Stange (16A) oder der Klemme (516A) beweglich ist, bevor die Vorrichtung zusammengesetzt wird.

11. Vorrichtung nach einem beliebigen der vorhergehenden Ansprüche 7 bis 10, **dadurch gekennzeichnet, dass** die Führungsöffnung (22A, 22B; 322A, 322B; 522A, 522B) eine längliche Form hat, wobei deren größte Abmessung sich in Richtung der Längsausdehnung der entsprechenden Schiene (12, 12'; 312; 512, 512'; 712_{SUP}, 712_{INF}) erstreckt.

12. Vorrichtung nach einem beliebigen der Ansprüche 1 bis 10, **dadurch gekennzeichnet, dass** die relative Führungsbahn (428B, 429B), wenn sie auf die horizontale Ebene der Wirbelsäule projiziert wird, zwischen den Verbindungsmitteln (412) und jeder der damit verbundenen wirbelseitigen Baugruppen eine Komponente (429B) aufweist, die ungleich null ist.

13. Vorrichtung nach Anspruch 12, **dadurch gekennzeichnet, dass** die Verbindungsmittel (412) und jede der verbundenen wirbelseitigen Baugruppen dazu geeignet sind, sich an jeweils mindestens zwei relativen Führungsflächen (430B₁, 412₁, 426B₁, 412₂) gegeneinander zu verschieben, wobei die Flächen im Wesentlichen ein und demselben konkaven Kugelabschnitt entsprechen, welcher der Wirbelsäule zugewandt ist.

14. Vorrichtung nach einem beliebigen der vorhergehenden Ansprüche, **dadurch gekennzeichnet, dass** die relativen Führungsbahnen zwischen den Verbindungsmitteln und den beiden verbundenen wirbelseitigen Baugruppen jeweils einen eindeutig bestimmbaren Mittelpunkt aufweisen.
